# EUROPEAN PATENT SPECIFICATION

(11) **EP 3 274 321 B1**
(45) Date of publication and mention of the grant of the patent: **18.09.2019**
(21) Application number: 16720930.3
(22) Date of filing: 22.03.2016
(51) Int. Cl.: C07C 37/70, C07C 39/23, A61K 31/05

(54) **CANNABIDIOL ISOLATE FROM INDUSTRIAL-HEMP AND USE THEREOF IN PHARMACEUTICAL AND/OR COSMETIC PREPARATIONS**
CANNABIDIOLISOLAT AUS INDUSTRIEHANF UND VERWENDUNG DAVON IN PHARMAZEUTISCHEN UND/ODER KOSMETISCHEN ZUBEREITUNGEN
ISOLAT DE CANNABIDIOL PROVENANT DE CHANVRE INDUSTRIEL ET UTILISATION DE CELUI-CI DANS DES PRÉPARATIONS PHARMACEUTIQUES ET/OU COSMÉTIQUES

(30) Priority: 23.03.2015 EP 15160293
(43) Date of publication of application: 31.01.2018
(73) Proprietor: Echo Pharmaceuticals B.V., 1382 GS Weesp (NL)
(72) Inventor: EIROA MARTINEZ, Cristina Maria, 1094 EJ Amsterdam (NL); VAN HOUTEN, Dennis, 1525 PV Westknollendam (NL); FERNANDEZ CID, Maria Vanesa, 2012 KE Haarlem (NL); WOERLEE, Geert Feye, 2012 LG Haarlem (NL)
(74) Representative: Nederlandsch Octrooibureau
(86) International application number: PCT/NL2016/050199
(87) International publication number: WO 2016/153347

(56) References cited:
- GB-A- 2 393 182
- US-A1- 2004 049 059

## Description

### Technical field of the invention

The present invention relates to a process for preparing cannabidiol isolates from industrial hemp, more in particular from industrial-hemp waste material.

### Background of the invention

Since many years cannabis (*Cannabis sativa*) has been used as a medicament for use in the treatment of various diseases and disorders. Cannabinoids, which are substituted monoterpenes, are the major active constituents of the plant *Cannabis sativa.* Despite various useful pharmacological properties, Cannabis has always been looked at with skepticism due to the abuse potential.

The principle cannabinoid components present in (herbal) cannabis are the cannabinoid acids Δ-9-tetrahydrocannabinolic acid (Δ9 THCA) and cannabidiolic acid (CBDA), with small amounts of the corresponding neutral cannabinoids, respectively Δ-9-tetrahydrocannabinol (Δ9 THC) and cannabidiol (CBD).

For quite some time, cannabidiol (and the corresponding acid) was regarded as inactive constituent, but there is now emerging evidence that CBD has pharmacological activity of its own that is quite distinct from that of Δ9 THC in several respects. CBD does not bind to the known cannabinoid receptors CB₁ or CB₂, and therefore does not cause the central or peripheral effects mediated by these receptors, as does THC. While this makes CBD a poor choice for recreational users, it gives the chemical a significant advantage as a medicine, since health professionals prefer treatments with minimal side effects. Although CBD and THC act on different pathways of the body, they seem to have many of the same medical benefits. According to a 2013 review published in the British Journal of Clinical Pharmacology, studies have found CBD to possess antiemetic, anticonvulsant, antipsychotic, anti-inflammatory, anti-oxidant, anti-tumoral, anxiolytic and anti-depressant effects. CBD also possess an important anti-bacterial effect.

In order to fully exploit the pharmacological potential of CBD it will be essential to have highly pure cannabidiol preparations, especially without the psychoactive contaminants such as THC. Such preparations must be obtainable using methods that are easy, relatively inexpensive and capable of scale-up.

Synthetic forms of cannabidiol are commercially available (e.g. from Sigma Corp.). However the costs involved in synthetic methods are prohibitive for practical pharmaceutical application.

Hence, to date, the availability of CBD preparations still entirely depends on extraction from the natural source, i.e. cannabis plant material.

The methods known in the art for preparing such purified forms of cannabinoids are generally not very efficient. Because of this lack of efficiency relatively high amounts of plant material are consumed. On top of that, most of the methods proposed in the art are applied to plant material obtained from cannabis cultivars that are high in cannabinoid levels (e.g. up to 20 % of THC(a) and around 10 % of CBD(a)). The procurement of such plant material is hampered due to regulatory constraints that are imposed in view of the potential for lucrative uses.

In the art, it has been suggested to use industrial hemp for the production of cannabinoid extracts. Industrial hemp has many uses, including paper, textiles, biodegradable plastics, construction, health food, animal food and fuel.

Hemp pellets are produced from the shiv, which is the woody core of the plant. The fiber is first separated and goes to make clothing and other products. The large shiv particles can then be used in construction in combination with lime. After all this processing has taken place there are small shiv particles remaining which can be processed into hemp pellets.

Industrial hemp has attractiveness as a source of CBD because it is available in huge amounts, as a waste product from various industries. At the same time, because of the relatively low content of cannabinoids, the use of industrial hemp poses additional challenges in making the extraction process economically viable.

For example, the purification process described in patent application GB 2393182, which is taught to be useful for the isolation of highly pure CBD, is not suited for the extraction of CBD from industrial hemp. As further demonstrated herein in Comparative Example 10 this process, which has been developed to derive CBD from cannabis plants having a "relatively high content of CBD" (see GB 2393182 at page 5, lines 14 to 20), results in such a low yield when used on industrial hemp material that no CBD crystals can actually be obtained.

This is due to the fact that while cannabis plants such as those used in patent application GB 2393182 usually have a CBD content of 5% or above, and even of 8% or above (by weight of dry matter), industrial hemp material typically has a CBD content of less than 5%, and even as low as less than 2%, by weight of dry matter. In the case of industrial hemp waste material, the CBD content can be as low as less than 0.5%, or even less than 0.3%, by weight of dry matter.

In US 2004/0049059 it is described to produce an extract containing THC and/or CBD from industrial hemp using a process that comprises the following steps:
- extraction with supercritical CO₂ (60°C, 250 bar);
- decarboxylation (80°C, 2 hours); and
- separation in a high pressure column (using CO₂ as solvent).
The method is shown to yield an extract containing CBD in approximately 90 % purity, which is insufficient to meet purity standards in pharmaceutical practice.

Hence, a need remains for a method for obtaining a high purity CBD extract from industrial hemp in good yield, which method is relatively simple, safe and inexpensive to carry out and which method is easily scalable. Furthermore, a need exists for a method that yields a well-defined extract in which the levels of CBD vary within narrow boundaries, so that it is useful as an ingredient in pharmaceutical and/or cosmetic formulations.

It is an object of the invention to provide a process that meets some or all of these objectives.

### Summary of the invention

The present inventors have developed a process for the isolation of highly pure cannabidiol (CBD) from industrial hemp material, preferably industrial hemp waste material, said process comprising:
a) providing industrial hemp material, preferably industrial hemp waste material, having a total content of Δ9-tetrahydrocannabinol (THC) and Δ9-tetrahydrocannabinolic acid (THCA) of less than 0.5 % by weight of dry matter and a total content of cannabidiol (CBD) and cannabidiolic acid (CBDA) of more than 0.2 %, preferably of more than 0.5 %, by weight of dry matter;
b) contacting the industrial hemp material with supercritical CO₂ at a temperature of 40-140 °C and a pressure of 100-400 bar to produce a crude CBD extract;
c) subjecting the crude CBD extract to thin film evaporation at a temperature of 100-180 °C and a pressure of 2^{∗}10⁻¹-1^{∗}10⁻³ mbar to produce a refined CBD extract;
d) dissolving the refined CBD extract in an organic solvent to produce a CBD solution, said organic solvent being selected from C₄₋₈ alkanes and combinations thereof;
e) inducing crystallization of dissolved CBD to form CBD crystals; and
f) collecting the CBD crystals and removing any trace of residual solvents;
wherein CBDA is decarboxylated to CBD prior to step c) by heating the industrial hemp material or the crude CBD extract to a temperature of 80-140 °C for at least 60 minutes.

The process of the invention allows for the use of industrial hemp (waste) as the starting material in an economically feasible manner. The process does not involve the use of chemicals that pose a health risk. Furthermore, the method involves the use of equipment that is relatively easy to implement, relatively inexpensive and that allows for scale-up without substantial difficulties.

The isolation method of the present invention offers the advantage that it yields a high purity CBD extract in good yield. The method further offers the advantage that it is highly reproducible in that it produces CBD isolate with a specific cannabinoid profile. More particularly, the method yields a CBD isolate that contains at least 95.0 % CBD by weight of dry matter (w/w) and less than 5 %, preferably less than 4%, more preferably less than 3%, of other cannabinoids combined, in weight of dry matter (w/w).

### Brief description of the Figures

Figure 1 illustrates the flash chromatography purification of the CBD distillate as described in Example 3. Figure 1 is a chromatogram (UV absorbance relative to mobile phase over time) of the purification, showing the fractions that have been produced (1-21).
Figure 2 illustrates the results obtained by purification of CBD fractions by crystallization (Example 4). The CBD peak is positioned at 6 minutes.
Figure 3 shows the chromatogram of the CBD crystals obtained by purification of a CBD isolate (Example 5).
Figure 4 shows the chromatogram of the CBD crystals obtained by purification of a CBD isolate (Example 7).
Figures 5 and 6 illustrate the stability of the purity in CBD on a weight basis (Figure 5) and on the basis of the HPLC peak area (Figure 6), for the CBD oils made with olive oil and sunflower oil, during 6 months of storage at room temperature and at 40°C (Example 9).

### Definitions

The term "cannabinoid" or "cannabinoids" as used herein encompasses at least the following substances: Δ-8 tetrahydrocannabinol, Δ-9-tetrahydrocannabinol (THC), cannabinol (CBN), cannabidiol (CBD), cannabigerol (CBG), Δ-9(11)-tetrahydrocannabinol (exo-THC), cannabichromene (CBC), tetrahydrocannabinol-C3 (THC-C3), tetrahydrocannabinol-C4 (THC-C4).

The term "cannabinoid acid", refers to the acid form of the above mentioned cannabinoids.

The abbreviation 'CBDA' is used herein to refer to cannabidiolic acid, which is the acid form of cannabidiol (CBD).

For ease of reference, the abbreviation CBD(a) may be used to refer to the combination of cannabidiol and cannabidiolic acid. Similarly, the term THC(a) may be used to refer to the combination of Δ-9-tetrahydrocannabinol and Δ-9-tetrahydrocannabinolic acid.

The term "Cannabis plant(s)" refers to wild type Cannabis sativa and also variants thereof, including cannabis chemovars (varieties characterized by means of chemical composition) which naturally contain different amounts of the individual cannabinoids, also *Cannabis sativa* subspecies indica including the variants *var. indica* and *var. kafiristanica,* Cannabis *indica* and also plants which are the result of genetic crosses, self-crosses or hybrids thereof.

The term "Cannabis plant material" encompasses a plant or plant part, e.g. bark, wood, leaves, stems, roots, flowers, seeds or parts thereof.

The term "hemp" is the common name for plants of the entire genus *Cannabis.*

The term "industrial hemp" as used herein refers to *Cannabis* strains cultivated for industrial use, i.e. any use except medicinal and/or recreational use, mostly as a source of fiber. Typically industrial hemp cultivars contain less than 0.5 % or less than 0.3 % of THC, based on the dry plant material. *Cannabis sativa L. subsp. sativa var. sativa* is the variety of hemp plant typically grown for industrial hemp production. Additionally, industrial hemp material typically contains less than 5%, or even less than 2% of CBD, by weight of dry matter. In the case of industrial hemp waste material, the CBD content can be as low as less than 0.5%, or even less than 0.3%, by weight of dry matter.

The term 'industrial hemp waste material' refers to the biomass remaining after processing of the industrial hemp, typically by extracting the hemp fiber.

The terms "decarboxylation treatment", "decarboxylation", "decarboxylated", as used herein, refer to a process step wherein Cannabis plant material has been treated such that the cannabinoid acids present in the untreated Cannabis plant material have been transformed into the corresponding neutral cannabinoids. In accordance with this invention, decarboxylation is typically carried out by heating the Cannabis plant material or the crude CBD extract.

The term "winterization" as used herein refers to a process which involves the chilling of a solvent extract of Cannabis plant material below 0° C for removal of, amongst others, fats and waxes, optionally combined with filtration and/or centrifugation.

The term "thin film evaporation" as used herein has its normal scientific meaning and refers to a method of evaporation wherein the mixture to be evaporated flows down the (heated) walls of an evaporator as a film.

The term "wiped film evaporation" as used herein has its normal scientific meaning and refers to a method of evaporation wherein the mixture to be evaporated is spread by wiper blades on the (heated) walls of an evaporator as a film.

The term "flash chromatography" as used herein has its normal scientific meaning as described amongst others in Still et al., in J. Org. Chem. Vol. 43, No. 14., 1978.

The term "carrier" or "pharmaceutically acceptable carrier" refers to a diluent, adjuvant, excipient, or vehicle with which a therapeutic agent, i.e. the CBD isolate, is administered.

"About" as used herein referring to a measurable value such as a parameter, an amount, a temporal duration, and the like, is meant to encompass variations of +/-10% or less, more preferably +/-5% or less, even more preferably +/-1% or less of and from the specified value, in so far such variations are appropriate to perform in the disclosed invention. However, it is to be understood that the value to which the modifier "about" refers is itself also specifically disclosed.

### Detailed description of the invention

A first aspect of the invention relates to a process for the isolation of highly pure cannabidiol (CBD) from industrial hemp material, preferably industrial hemp waste material, said process comprising:
a) providing industrial hemp material, preferably industrial hemp waste material, having a total content of THC and THCA of less than 0.5 % by weight of dry matter and a total content of CBD and CBDA of more than 0.2 %, preferably of more than 0.5 %, by weight of dry matter;
b) contacting the industrial hemp material with supercritical CO₂ at a temperature of 40-140 °C and a pressure of 100-400 bar to produce a crude CBD extract;
c) subjecting the crude CBD extract to thin film evaporation at a temperature of 100-180 °C and a pressure of 2^{∗}10⁻¹-1^{∗}10⁻³ mbar to produce a refined CDB extract;
d) dissolving the refined CBD extract with an organic solvent to produce a CBD solution, said organic solvent being selected from C₄₋₈ alkanes and combinations thereof;
e) inducing crystallization of dissolved CBD to form CBD crystals; and
f) collecting the CBD crystals and removing any trace of residual solvents;
wherein CBDA is decarboxylated to CBD prior to step c) by heating the hemp material or the crude CBD extract to a temperature of 80-140 °C for at least 60 minutes .

Preferably, the industrial hemp material comprises fresh or processed plant material obtained from industrial hemp, preferably *Cannabis sativa L. subsp. sativa var. sativa.* Suitable varieties include for example Fedora 19, Felina 45, Futura 77, Futura 75, Kompolti, Uniko-B, Finola 314, etc.

The total THC(a) content of the industrial hemp material preferably is below 0.4 % by weight of dry matter, more preferably below 0.3 % and most preferably below 0.2 %. The total CBD(a) content of the industrial hemp material preferably is more than 1% by weight of dry matter, most preferably more than 1.5 % by weight of dry matter.

Industrial hemp material suitable for use in accordance with the invention includes fresh and/or processed plant parts selected from flowers, leaves, bark, wood, stalks and combinations thereof. As noted before, it is particularly preferred in accordance with the invention, that the starting material is industrial hemp waste material, i.e. plant parts, such as those recited here before, which have been subjected to treatments aimed at procuring plant components other than the cannabinoids, such as, in particular, fiber. Hence, in one particularly preferred embodiment of the invention, step a) of the process comprises providing an industrial hemp material that remains (as waste) after fiber extraction from e.g. fresh or dried hemp straw material. Hence, in an embodiment of the invention, the industrial hemp waste material comprises hemp straw characterized by a relatively low fiber content, e.g. hemp straw material comprising less than 90 kg of fiber per 100 kg of hemp straw material, based on dry weight, more preferably less than 80 kg, per 100 kg of hemp straw material, most preferably less than 70 kg per 100 kg of hemp straw material. In an embodiment of the invention, the industrial hemp waste material comprises hemp straw material characterized by a CBD(a) to fiber ratio that is significantly higher than that of unprocessed hemp straw, e.g. at least 10 g of CBD(a) per kg of hemp straw material, preferably at least 20 g of CBD(a) per kg of hemp straw material, most preferably at least 30 g CBD(a) per kg of hemp straw material.

In a preferred embodiment of the invention, the industrial hemp (waste) material is subjected to a treatment resulting in a reduction of the particle size, such as cutting or grinding.

The extraction step comprises contacting the industrial hemp with supercritical CO₂. Typically, the hemp material is placed in a pressure vessel following which supercritical CO₂ is passed into the vessel. The supercritical CO₂ is preferably at a temperature of 35-120°C, most preferably at a temperature of 40-100°C. In the process of the invention, the supercritical CO₂ is preferably at a pressure of 100-350 bar, most preferably at a pressure of 150-300 bar.

In order to obtain the crude extract, CO₂ containing dissolved compounds is typically collected in a vessel where the pressure is lowered so that the dissolved compounds precipitate to form the crude extract. Typically, for this purpose the CO₂ containing dissolved compounds is transported to a separator vessel, where the pressure is lowered and the dissolved compounds precipitate to produce the crude CBD extract.

Since the industrial hemp material from which CBD is to be prepared typically contains significant amounts of the cannabidiol acid CBDA, the plant material or crude extract is subjected to a decarboxylation step to convert CBDA to CBD. Decarboxylation is preferably carried out by heating the CBD(a) containing material to a temperature within the range of 90-130°C, more preferably within the range of 100-120°C. Decarboxylation of cannabinoid acids is a function of time and temperature and the person skilled in the art is capable to determine the appropriate amount of time necessary for complete conversion. In a preferred embodiment of the invention, the decarboxylation step is carried out by heating the precipitate collected in the separator vessel to the temperatures recited above, typically for a period of time within ranging from 0.5-8 hours, preferably 0.5-2 hours.

The crude CBD extract is subjected to thin film evaporation to produce a refined extract. Thin film evaporation is preferably performed at a temperature within the range of 100-160°C, more preferably within the range of 120-140°C. Preferably, thin film evaporation is performed at a pressure within the range of 8^{∗}10⁻²-1^{∗}10⁻³ mbar, preferably within the range of 6^{∗}10⁻²-1^{∗}10⁻³ mbar. In a preferred embodiment of the present invention, a wiped film evaporator (WFE) is used, more preferably a short path wiped film evaporator.

The skilled artisan would know how to fine tune the WFE parameters in order to optimize output values such as % CBD in the output stream, or in order to obtain a suitable colour for the output stream (a golden orange colour for the resin is indicative of a better CBD output quality than a darker, such as black, resin).

During the thin film evaporation step as much non-cannabinoid compounds as possible are separated from the crude solvent extract. Typically, the amount of non-cannabinoid compounds in the refined extract is reduced to less than 50 wt.%, even more preferably to less than 40 wt.% and most preferably to less than 30 wt.% in this thin film evaporation step.

According to a particularly preferred embodiment of the present method the crude extract is first subjected to rotary evaporation, i.e. until a relatively viscous composition is obtained, and subsequently to thin film evaporation, preferably wiped film evaporation, so as to obtain the refined extract.

The refined CBD extract is then preferably subjected to crystallization. This step comprises dissolving the refined CBD extract in an organic solvent, said organic solvent being selected from C₄₋₈ alkanes and mixtures thereof. In a preferred embodiment of the invention, the solvent is pentane. The amount of solvent used is not critical. For practical reasons it is preferred to use the minimum amount necessary to dissolve the refined extract. In a preferred embodiment the solvent is heated prior to and/or during the step of combining it with the refined CBD extract, e.g. to a temperature within the range of 20-60°C, preferably 25-50°C, more preferably 30-45°C. The CBD solution is subsequently cooled to induce crystallization of the CBD. Typically, for that purpose, the CBD solution is chilled and kept at a temperature below 0°C, preferably within the range of -20 to 0°C, more preferably within the range of -20 to -10°C. The exact period of time required for complete crystallization of the CBD will depend on the exact conditions. Typically, in accordance with the invention, the CBD solution is kept under the temperatures recited above for a period of at least 12 hours, preferably for a period of 12-60 hours, more preferably for a period of 24-48 hours.

Following crystallization, the CBD crystals are collected, e.g. by filtration. In order to further enhance the efficiency of the process of the invention, the filtrate is not discarded but subjected to a further crystallization step.

The process of the invention may comprise one or more steps of washing the CBD crystals obtained in step f), each washing step comprising combining the crystals with a suitable liquid, e.g. n-pentane, and filtering to remove insoluble material. Solvent is then removed, e.g. by rotary evaporation, to produce crystalline CBD. By way of illustration, rotary evaporation may be performed for about 3 hours or more, at about 40°C. All steps are carried out according to standard laboratory procedures, such as would be known to those skilled in the art.

The collected crystals are subjected to a milling process to homogenise the particle size and subsequently to rotary evaporation to eliminate any residual solvent.

The milled and dried crystals are suitable for incorporation in pharmaceutical, cosmetic and/or dermatological formulations, as will be explained in some more detail here below.

In certain embodiments of the invention, the process comprises the additional step of subjecting the refined extract obtained in step c) to winterization or chromatography, where after the crystallization process according to steps d)-e) as described here above is performed on the resulting highly refined CBD fraction(s).

Winterization is typically aimed at the removal of waxy components, which is typically accomplished by dissolving the refined extract in an organic solvent, said solvent being selected from the group consisting of methanol, ethanol, other alkyl alcohols and mixtures thereof. The use of methanol is particularly preferred. The CBD-containing solution thus obtained is then typically chilled and kept at a temperature of below 0°C, preferably within the range of -30 to -5°C, more preferably within the range of -25 to -10°C, which causes precipitation of the waxy components. Typically, in accordance with the invention, the CBD solution is kept under the temperatures recited above for a period of at least 10 hours, preferably for a period of 12-60 hours, more preferably for a period of 24-48 hours. Following chilling, the obtained liquid is subjected to a treatment resulting in separation of a precipitate and a solution. For example, the liquid may be subjected to centrifugation and/or filtration. By way of illustration, centrifugation may be performed for about 10 mins at about 6000 rpm and at about 0°C. The CBD containing solution thus obtained is typically subjected to evaporation, preferably by thin film evaporation, more preferably wiped film evaporation, before subjecting it to the crystallization process of steps d) and e). Preferably, evaporation is applied to reduce the amount of solvent to less than 0.5 wt.%, even more preferably to less than 0.3. wt.% and most preferably to less than 0.03 wt.%.

In accordance with the invention, the preferred chromatographical fractionation process is flash chromatography. The chromatographical fractionation is preferably done using a mixture of C5-C8 alkane and ethylacetate as eluent. Such a mixture comprises preferably 1-10 vol% ethylacetate, most preferably 1-5 vol% ethylacetate. According to a particularly preferred embodiment, the eluent employed is a mixture of C5-C6 alkane and ethyl acetate. Most preferably, the eluent is a mixture of hexane and ethyl acetate. In accordance with the invention, the stationary phase for the chromatographical fractionation process is selected from the group consisting of silica, silica gel and Silica xerogel. Most preferably amorphous synthetic silica gel is used as the stationary phase.

In order to further enhance the efficiency (e.g. yield) of the method according to the present invention the one or more low purity fractions produced in the chromatographical fractionation step are combined with the crude solvent extract and/or the refined extract. Most preferably, the one or more low purity fractions are evaporated and combined with the crude solvent extract. According to the present invention, the high purity fraction obtained in the chromatographical fractionation step is subjected to a second or further thin film evaporation step, preferably to a rotary evaporation. In this step the solvents used in the chromatographical fractionation step are substantially removed.

CBD-containing high purity fractions thus obtained are typically subjected to evaporation, preferably by thin film evaporation, more preferably wiped film evaporation, before subjecting them to the crystallization process of steps d) and e). Preferably, evaporation is applied to reduce the amount of solvent to less than 0.5 wt.%, even more preferably to less than 0.3. wt.% and most preferably to less than 0.03 wt.%.

In order to further increase the efficiency of the present method it is preferred to first subject the high purity fractions obtained from the chromatographical fractionation step to rotary evaporation.

Even though the present method is highly effective in separating CBD from the other cannabinoids present in the industrial hemp (waste) material, some traces of the other cannabinoids typically found in the industrial hemp may remain in the CBD isolate of the present invention. Hence, in some embodiments, the CBD isolate is characterized by the presence of at least one, at least two, at least three, at least four, at least five or at least six components selected from c) - i) as defined above.

In an embodiment of the invention, a CBD isolate obtained using the process of the invention comprises 0.001 - 0.1 % Δ8-THC by weight of dry matter, more preferably 0.002 - 0.05 %, most preferably 0.005 - 0.01 %.

In an embodiment of the invention, a CBD isolate obtained using the process of the invention comprises 0.001 - 0.5% CBC by weight of dry matter, more preferably 0.005 - 0.1 %, most preferably 0.01 - 0.05 %.

In an embodiment of the invention, a CBD isolate obtained using the process of the invention comprises 0.001 - 0.1 % CBN by weight of dry matter, more preferably 0.002 - 0.05 %, most preferably 0.005 - 0.01 % .

In an embodiment of the invention, a CBD isolate obtained using the process of the invention comprises 0.001 - 0.1 % exo-THC by weight of dry matter, more preferably 0.002 - 0.05 %, most preferably 0.005 - 0.01 %.

In an embodiment of the invention, a CBD isolate obtained using the process of the invention comprises 0.001 - 0.1 % CBG by weight of dry matter, more preferably 0.002 - 0.05 %, most preferably 0.005 - 0.01 %.

In an embodiment of the invention, a CBD isolate obtained using the process of the invention comprises 0.001 - 0.1 % THC-C3 by weight of dry matter, more preferably 0.002 - 0.05 %, most preferably 0.005 - 0.01 %.

In an embodiment of the invention, a CBD isolate obtained using the process of the invention comprises 0.001 - 0.1 % THC-C4 by weight of dry matter, more preferably 0.002 - 0.05 %, most preferably 0.005 - 0.01 % .

In an embodiment of the invention, a CBD isolate obtained using the process of the invention comprises detectable amounts of CBD, of Δ9-THC, of Δ8-THC, of CBC, of CBN and of THC-C4.

In an embodiment of the invention, a CBD isolate obtained using the process of the invention comprises detectable amounts of CBD, of Δ9-THC, of Δ8-THC, of CBC, and of THC-C4.

In an embodiment of the invention, a CBD isolate obtained using the process of the invention comprises the following compounds in the following amounts, as measured on the basis of the HPLC peak areas:

| ***Compound*** | ***% by area*** |
|---|---|
| CBD | about 99.6 |
| Δ9-THC | about 0.04 |
| Δ8-THC | less than 0.05 |
| CBC | about 0.03 |
| CBN | about 0.01 |
| THC-C4 | less than 0.05 |

In an embodiment of the invention, a CBD isolate obtained using the process of the invention comprises the following compounds in the following amounts, as measured on the basis of the HPLC peak areas:

| ***Compound*** | ***% by area*** |
|---|---|
| CBD | about 97.75 |
| Δ9-THC | about 0.02 |
| Δ8-THC | less than 0.05 |
| CBC | about 0.03 |
| THC-C4 | less than 0.05 |

In an embodiment of the invention, a CBD isolate obtained using the process of the invention comprises the following compounds in the following amounts, as measured on the basis of the HPLC peak areas:

| ***Compound*** | ***% by area*** |
|---|---|
| CBD | about 96.76 |
| Δ9-THC | about 0.02 |
| Δ8-THC | less than 0.05 |
| CBC | about 0.01 |
| THC-C4 | less than 0.05 |

In an embodiment of the invention, a CBD isolate obtained using the process of the invention comprises the compounds reported in one of the profiles selected from those listed in Table 2, in Table 3 or in Table 4 of the Examples, in about the amounts reported in said tables, including the listed impurities at the given retention times.

Preferably, the residual amount of residual solvents in the CBD isolate of the present invention is below 10.000 ppm, more preferably below 5000 ppm. This way a product is obtained wherein the amount of solvents is so low that it does not pose a health risk.

The invention will now be illustrated by way of the following, non-limiting examples.

### Examples

### Example 1: production of CBD extract

Hemp material is obtained from industrial hemp waste pellets, 200 g of this material is placed in an extraction vessel. Extraction is performed using CO₂ at a temperature of 60 °C and pressure of 300 bar that is pumped over the plant material in the extraction vessel. CO₂ is transported from the extraction vessel to a separator vessel. In the separator vessel the CO₂ is brought to a temperature of 50-60 °C and a pressure of 59-60 bar, or other as necessary to precipitate the compounds. A precipitate is formed in the separator vessel. After 6 hours the CO₂ supply is stopped. The pressure is reduced to atmospheric conditions and the temperature is reduced to room temperature. The sample is collected. The conditions applied during CO₂ extraction are summarized in table 1.

**Table 1 CO₂ extraction**

| ***Parameter*** | **Set conditions** |
|---|---|
| Temperature extraction vessel | 60 °C |
| Pressure extraction vessel | 300 bar |
| CO₂ flow | 9.5 kg/h |
| Extraction time | 2 h |
| Batch size | 200 g plant material |

The sample is subjected to decarboxylation treatment while still contained in the separator vessel, by heating to 120°C for 1 hour. A black resin containing 20 % CBD extract is obtained.

### Example 2: Purification of CBD extract by distillation

The CBD extract obtained as described in example 1 can be subjected to several purification steps. This material is distilled using a Short Path Wiped Film Evaporator (WFE). The main cannabinoids are separated from the non-cannabinoids. The collected cannabinoid fraction is weighed and analysed.

The CBD distillate obtained after WFE treatment has a percentage by weight of approximately 60 %.

### Example 3: Purification of CBD distillate by Chromatography

The CBD isolate produced as example 2 is used for further purification. The purification is performed by chromatographic separation with flash chromatography. The mobile phase used in the column is hexane with 3 % of ethyl acetate. The stationary phase used is silica.

Several fractions obtained from the flash chromatography are analyzed by HPLC and the fractions with similar purity are combined.

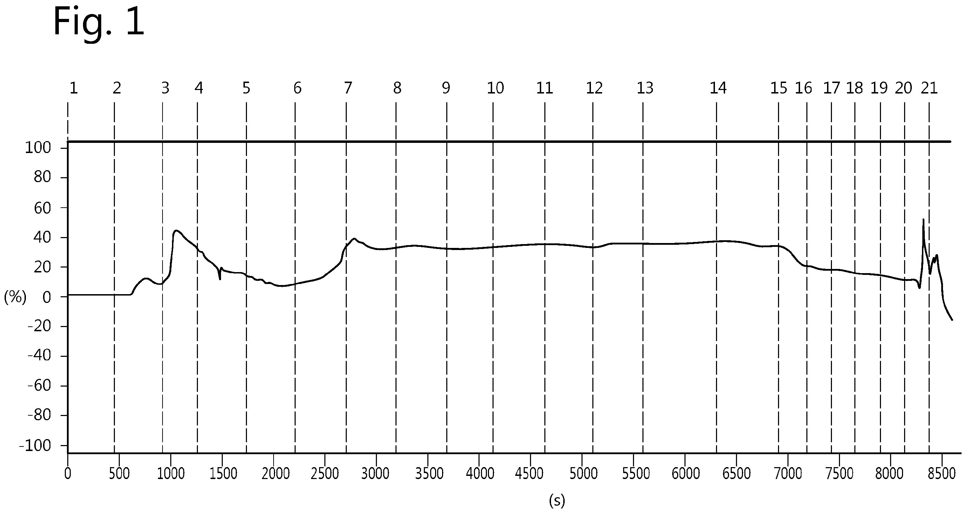

Figure 1 is a chromatogram (UV absorbance relative to mobile phase over time) of the purification, showing the fractions that have been produced (1-21). The main fractions obtained that have high CBD content are combined together and subjected to Rotavapor evaporation. This isolate contains high CBD percentage (≥ 90 %).

### Example 4: Purification of CBD combined fraction by Crystallization

The CBD isolate created as described in example 3 is subjected to a crystallization step by dissolution of the combined fraction in the minimal amount of warm pentane. The sample is introduced in the freezer during 24 h to induce the crystallization. The sample is filtrated and the obtained crystals are then dried by evaporation of the solvents in a Rotavapor. The temperature used in the drying is 40 °C at continuous vacuum during at least 3 hours.

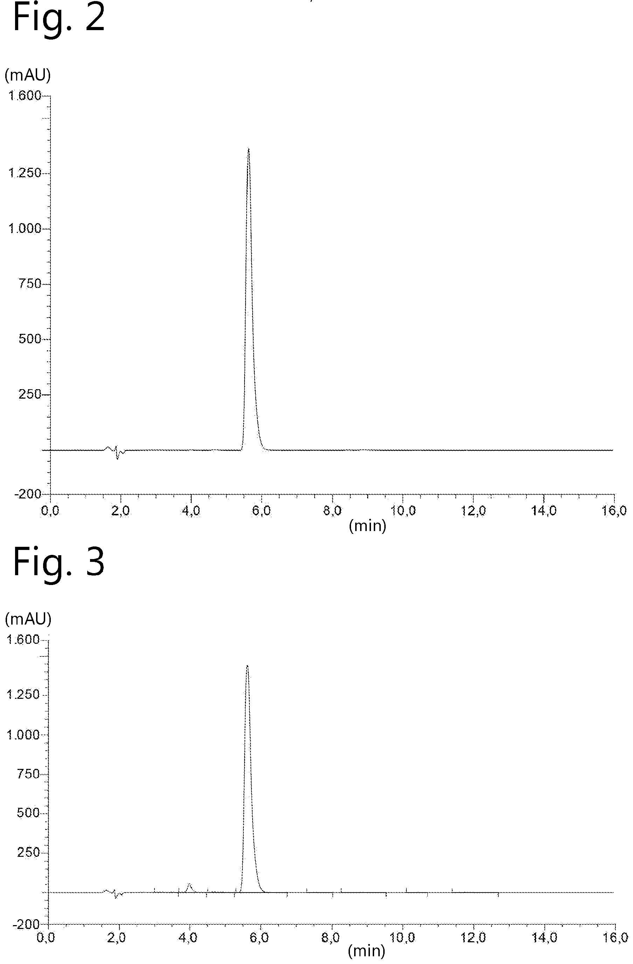

The obtained crystals created using the method with flash chromatography have a high purity > 99.5%. Table 2 show the impurities present in the final CBD crystals. Figure 2 illustrates the results obtained for example 4. The CBD peak is positioned at 6 minutes.

**Table 2 Purity profile CBD crystals example 4**

| ***Compound*** | ***% by area*** |
|---|---|
| CBD | 99.60 |
| Δ9-THC | 0.04 |
| Δ8-THC | <0.05 |
| CBC | 0.03 |
| CBN | 0.01 |
| exo-THC | n.d. |
| CBG | n.d. |
| THC-C3 | n.d. |
| THC-C4 | <0.05 |
| Imp 1 (RRT 0.56) | n.d. |
| Imp 2 (RRT 0.71) | 0.03 |
| Imp 3 (RRT 0.83) | 0.11 |
| Imp 4 (RRT 1.33) | 0.03 |
| Imp 5 (RRT 1.58) | 0.14 |
| Imp 6 (RRT 1.62) | n.d. |
| Imp 7 (RRT 2,09) | n.d. |

| | |
|---|---|
| *n.d.: not detected* | |

### Example 5: Purification of CBD distillate by Crystallization

A CBD isolate produced as explained in example 2 is subjected to crystallization performed by dissolving the sample in the minimum amount of warm pentane (40°C) possible. After that the sample is cooled in a water-ice bath and introduced in the freezer at least 24 h. The crystals obtained are filtered and washed. The crystals are then dried by evaporation of the solvents in a Rotavapor. The temperature used in the drying is 40 °C at continuous vacuum during at least 3 hours.

The obtained crystals created with crystallization after WFE have a purity > 95 %. A chromatogram of the obtained CBD crystals can be seen in figure 3 and table 3 shows the impurities present in the final CBD crystals.

**Table 3 Purity profile CBD crystals example 5**

| ***Compound*** | ***% by area*** |
|---|---|
| CBD | 97.75 |
| Δ9-THC | 0.02 |
| Δ8-THC | <0.05 |
| CBC | 0.03 |
| CBN | n.d. |
| exo-THC | n.d. |
| CBG | n.d. |
| THC-C3 | n.d. |
| THC-C4 | <0.05 |
| Imp 1 (RRT 0.56) | n.d. |
| Imp 2 (RRT 0.71) | 1.62 |
| Imp 3 (RRT 0.83) | 0.15 |
| Imp 4 (RRT 1.33) | 0.08 |
| Imp 5 (RRT 1.58) | 0.09 |
| Imp 6 (RRT 1.62) | 0.04 |
| Imp 7 (RRT 2,09) | 0.21 |

| | |
|---|---|
| *n.d.: not detected* | |

### Example 6: Purification of CBD distillate by Winterization

The sample after WFE as described in example 2 was dissolved in MeOH and introduced in the freezer at -20°C during 48 hours. After that the sample was centrifuged at 6000 rpm at 0°C during 10 minutes to eliminate the waxes. After centrifugation the sample was filtrated and evaporated.

### Example 7: Purification of CBD sample after winterization by Crystallization

The CBD isolate obtained in example 6 was subjected to a crystallization step. The sample is dissolved in the minimal amount of warm pentane. The sample is introduced in the freezer during 24 h to induce the crystallization. The crystals are then dried by evaporation of the solvents in a Rotavapor. The temperature used in the drying is 40°C at continuous vacuum at least 3 hours.

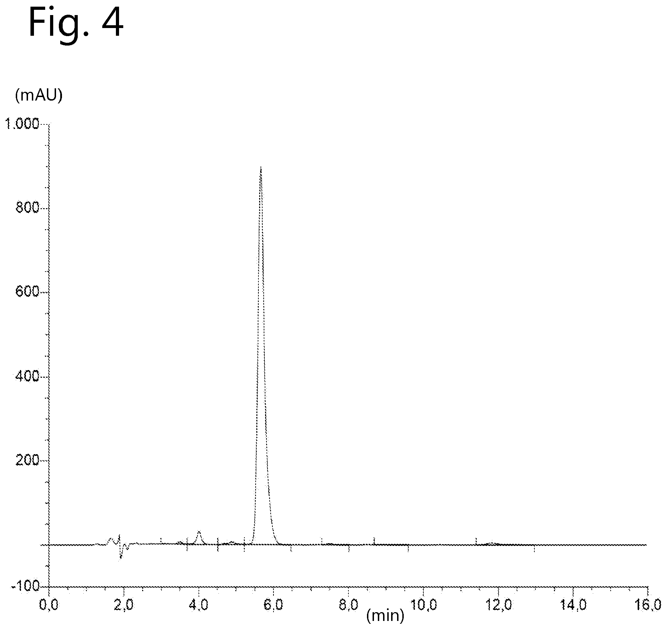

The obtained crystals after WFE, winterization and crystallization have a purity > 95 %. A chromatogram of the obtained CBD crystals can be seen in figure 4 and table 4 shows the impurities present in the final CBD crystals.

**Table 4 Purity profile CBD crystals example 7**

| ***Compound*** | **% *by area*** |
|---|---|
| CBD | 96.76 |
| Δ9-THC | 0.02 |
| Δ8-THC | <0.05 |
| CBC | 0.01 |
| CBN | n.d. |
| exo-THC | n.d. |
| CBG | n.d. |
| THC-C3 | n.d. |
| THC-C4 | <0.05 |
| Imp 1 (RRT 0.56) | 0.15 |
| Imp 2 (RRT 0.71) | 2.45 |
| Imp 3 (RRT 0.83) | 0.29 |
| Imp 4 (RRT 1.33) | 0.06 |
| Imp 5 (RRT 1.58) | 0.07 |
| Imp 6 (RRT 1.62) | n.d. |
| Imp 7 (RRT 2,09) | 0.18 |

| | |
|---|---|
| *n.d.: not detected* | |

### Example 8: Use of CBD isolate in a cream

A CBD isolate produced from hemp waste material was used as active ingredient for the creation of a cream. Pure CBD crystals are dispersed in a vegetable oil and emulsified into a nourishing and soothing base cream. Preparation of a 0.5 wt.% CBD cream is performed in two steps: solution of CBD in oil and emulsification of this CBD oil into the base cream. A solution of 17 wt.% CBD in sunflower oil is prepared by dissolving the CBD crystals in the vegetable oil at 40°C under magnetic stirring. Then the solution of CBD in oil is added to a base cream and emulsified with a laboratory mixer, such as T 25 digital ULTRA-TURRAX® from IKA, mixing with speed 3 during 10 minutes. Finally, the formulation is dispensed in the package.

The qualitative composition of the CBD cream is given in Table 5. Final cream contains 0.5 wt.% of natural pure CBD.

**Table 5 CBD-Cream composition**

| Pure and Natural CBD |
|---|
| Vegetable oil |
| Base Cream (Aqua, Petrolatum, Glycerin, Cetearyl Alcohol, Urea, Glyceryl Stearate Se, Stearic Acid, Dimethicone, Cyclopentasiloxane, Phenoxyethanol, PEG-40 Hydrogenated Castor Oil, Parfum, Caprylhydroxamic Acid, Methylpropanediol) |

### Example 9: Use of CBD isolate in oil preparations and stability studies

A CBD isolate produced from hemp waste material was used as active ingredient for the creation of CBD oils which may be used for cosmetic, pharmaceutical or nutritional purposes.

Pure CBD crystals are dissolved in different vegetable oils. Table 6 provides the % CBD content for each experiment.

**Table 6 Percentage CBD content for different oil preparations**

| ***Experiment*** | ***% CBD*** |
|---|---|
| CBD in olive oil | 36.2 |
| CBD in sunflower oil | 35.0 |
| CBD in rice oil | 33.2 |
| CBD in peanut oil | 35.4 |

Out of the four different CBD oils, two of them were subjected to a stability study at room temperature and at 40°C for up to 6 months.

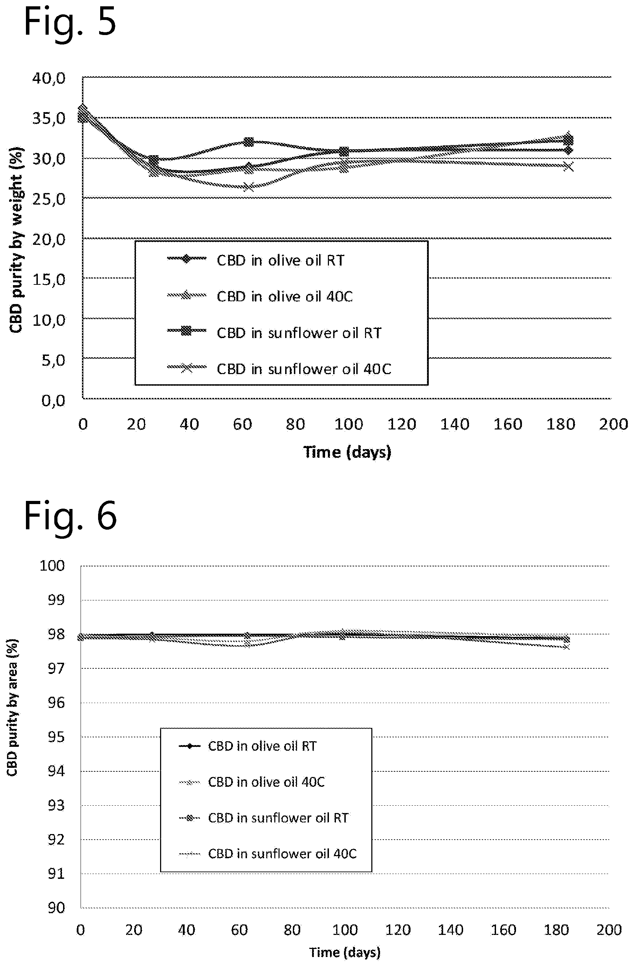

The stability of the purity in CBD was monitored on a weight basis (Figure 5) as well as on the basis of the HPLC peak area (Figure 6).

As can be observed in the Figures, satisfactory stability is observed for up to 6 months' time with the preparations using the CBD extract of the invention, making it a suitable source of supply for preparations in highly regulated environments, such as for pharmaceutical or cosmetic applications.

### Comparative Example 10: Application of a previously known method of preparing cannabidiol to Industrial Hemp material

The preparation process described in patent application GB 2393182 was applied to the industrial hemp material used in Example 1 above and, as a control, to a cannabis plant extract with a CBD content greater than 8% by weight of dry matter.

Briefly, the starting material is first submitted to a CO₂ extraction step and a decarboxylation step. Subsequently the extract is submitted to a winterization step and a charcoal clean-up step. Crystallisation is then performed using a suitable solvent which is subsequently evaporated to form the crystals.

In accordance with the teachings of GB 2393182, this process applied to the cannabis plant extract (control) is able to yield CBD crystals with greater than 98% purity.

By contrast, applying the same process to the industrial hemp material used in Example 1 above yields an extract containing only ca. 40% CBD, which is impossible to crystallize due to the concentration being too low.

This difference is easily understandable since the first step of the process (CO₂ extraction step) yields a CBD concentration of 55 to 60% when starting from cannabis plant extract, but of only 20 to 25% when starting from industrial hemp material.

In order to produce cosmetic or pharmaceutical grade CDB, the process of the prior art is therefore not suited to process industrial hemp material.

## Claims

1. A process for the isolation of highly pure cannabidiol (CBD) from industrial hemp material, preferably industrial hemp waste material, said process comprising:
a) providing industrial hemp material, preferably industrial hemp waste material, having a total content of Δ9-tetrahydrocannabinol (THC) and Δ9-tetrahydrocannabinolic acid (THCA) of less than 0.5 % by weight of dry matter and a total content of cannabidiol (CBD) and cannabidiolic acid (CBDA) of more than 0.2 % by weight of dry matter;
b) contacting the industrial hemp material with supercritical CO₂ at a temperature of 40-140 °C and a pressure of 100-400 bar to produce a crude CBD extract;
c) subjecting the crude CBD extract to thin film evaporation at a temperature 100-180°C and a pressure of 2^{∗}10⁻¹-1^{∗}10⁻³ mbar to produce a refined CBD extract;
d) dissolving the refined CBD extract in an organic solvent to produce a CBD solution, said organic solvent being selected from C₄₋₈ alkanes and combinations thereof;
e) inducing crystallization of dissolved CBD to form CBD crystals; and
f) collecting the CBD crystals and removing residual solvents
wherein CBDA is decarboxylated to CBD prior to step c) by heating the hemp material or the crude CBD extract to a temperature of 80-140 °C for at least 60 minutes.

2. Process according to claim 1, wherein in step c) the thin film evaporation is carried out by wiped film evaporation.

3. Process according to claim 1 or 2, wherein the wiped film evaporator used in step c) is a short path wiped film evaporator.

4. Process according to any one of the previous claims, wherein the crude extract obtained in step c) is subjected to chromatographical fractionation to produce a high purity CBD fraction.

5. Process according to claim 4, wherein the high purity CBD fraction is subjected to evaporation before subjecting it to the crystallization process comprising steps d) and e).

6. Process according to claim 4 or 5, wherein the chromatographical fractionation comprises flash chromatography, which is carried out with a hexane / ethylacetate mixture as eluent.

7. Process according to any one of the previous claims, wherein the crude extract obtained in step c) is subjected to winterization, to produce a CBD containing solution.

8. Process according to claim 7, wherein the CBD containing solution is subjected to evaporation before subjecting it to the crystallization process comprising steps d) and e).

9. Process according to any one of the previous claims, wherein the industrial hemp material or the industrial hemp waste material provided in step a) contains less than 5%, preferably less than 2%, of CBD by weight of dry matter.

## Patentansprüche

1. Verfahren zur Isolierung von hochreinem Cannabidiol (CBD) aus Industriehanfmaterial, vorzugsweise Industriehanf-Abfallmaterial, wobei das Verfahren umfasst:
(a) Bereitstellen von Industriehanfmaterial, vorzugsweise Industriehanf-Abfallmaterial, mit einem Gesamtgehalt an Δ9-Tetrahydrocannabinol (THC) und Δ9-Tetrahydrocannabinolsäure (THCA) von weniger als 0,5 % bezogen auf das Gewicht der Trockenmasse und einem Gesamtgehalt an Cannabidiol (CBD) und Cannabidiolsäure (CBDA) von mehr als 0,2 % bezogen auf das Gewicht der Trockenmasse;
(b) In-Kontakt-Bringen des Industriehanfmaterials mit superkritischem CO₂ bei einer Temperatur von 40 - 140 °C und einem Druck von 100 - 400 bar, um einen rohen CBD-Extrakt herzustellen;
(c) den rohen CBD-Extrakt einer Dünnfilmverdampfung bei einer Temperatur von 100 - 180 °C und einem Druck von 2 x 10⁻¹ - 1 x 10⁻³ mbar auszusetzen, um einen raffinierten CBD-Extrakt herzustellen;
(d) Lösen des raffinierten CBD-Extrakts in einem organischen Lösungsmittel, um eine CBD-Lösung herzustellen, wobei das organische Lösungsmittel aus C₄₋₈-Alkanen und Kombinationen davon ausgewählt ist;
(e) Induzieren der Kristallisation des gelösten CBD, um CBD-Kristalle zu bilden, und
(f) Gewinnen der CBD-Kristalle und Entfernen restlicher Lösungsmittel,
wobei CBDA vor Schritt c) durch mindestens 60 Minuten langes Erhitzen des Hanfmaterials oder des rohen CBD-Extrakts auf eine Temperatur von 80 - 140 °C zu CBD decarboxyliert wird.

2. Verfahren gemäß Anspruch 1, wobei in Schritt c) die Dünnfilmverdampfung durch Wischfilmverdampfung ausgeführt wird.

3. Verfahren gemäß Anspruch 1 oder 2, wobei es sich bei dem in Schritt c) verwendeten Wischfilmverdampfer um einen Kurzweg-Wischfilmverdampfer handelt.

4. Verfahren gemäß einem der vorherigen Ansprüche, wobei der in Schritt c) erhaltene Rohextrakt einer chromatographischen Fraktionierung unterzogen wird, um eine CBD-Fraktion hoher Reinheit herzustellen.

5. Verfahren gemäß Anspruch 4, wobei die CBD-Fraktion hoher Reinheit einer Verdampfung unterzogen wird, bevor sie dem Kristallisationsverfahren, das die Schritte d) und e) umfasst, unterzogen wird.

6. Verfahren gemäß Anspruch 4 oder 5, wobei die chromatographische Fraktionierung eine Blitz-Chromatographie umfasst, die mit einem Hexan/Ethylacetat-Gemisch als Elutionsmittel ausgeführt wird.

7. Verfahren gemäß einem der vorherigen Ansprüche, wobei der in Schritt c) erhaltene Rohextrakt einer Winterisierung unterzogen wird, um eine CBD-haltige Lösung herzustellen.

8. Verfahren gemäß Anspruch 7, wobei die CBD-haltige Lösung einer Verdampfung unterzogen wird, bevor sie dem Kristallisationsverfahren, das die Schritte d) und e) umfasst, unterzogen wird.

9. Verfahren gemäß einem der vorherigen Ansprüche, wobei das in Schritt a) bereitgestellte Industriehanfmaterial oder Industriehanf-Abfallmaterial weniger als 5 %, vorzugsweise weniger als 2 % CBD bezogen auf das Gewicht der Trockenmasse enthält.

## Revendications

1. Procédé pour isoler du cannabidiol (CBD) très pur à partir de chanvre industriel, de préférence de déchets de chanvre industriel, ladite procédé comprenant les étapes consistant à :
a) fournir du chanvre industriel, de préférence des déchets de chanvre industriel, ayant une teneur totale en Δ9-tétrahydrocannabinol (THC) et en acide Δ9-tétrahydrocannabinolique (THCA) inférieure à 0,5 % en poids de matière sèche et une teneur totale en cannabidiol (CBD) et en acide cannabidiolique (CBDA) supérieure à 0,2 % en poids de matière sèche ;
b) mettre en contact le chanvre industriel avec du CO₂ supercritique à une température de 40 à 140°C et à une pression de 100 à 400 bars pour produire un extrait de CBD brut ;
c) soumettre l'extrait de CBD brut à une évaporation en film mince à une température de 100 à 180°C et à une pression de 2^{∗}10⁻¹ à 1^{∗}10⁻³ mbar pour produire un extrait de CBD raffiné ;
d) dissoudre l'extrait de CBD raffiné dans un solvant organique pour produire une solution de CBD, ledit solvant organique étant choisi parmi des alcanes en C₄ à C₈ et des combinaisons de ceux-ci ;
e) induire une cristallisation du CBD dissous pour former des cristaux de CBD ; et
f) collecter les cristaux de CBD et éliminer des solvants résiduels
dans lequel le CBDA est décarboxylé en CBD avant l'étape c) en chauffant le chanvre ou l'extrait de CBD brut à une température de 80 à 140°C pendant au moins 60 minutes.

2. Procédé selon la revendication 1, dans lequel, à l'étape c), l'évaporation en film mince est réalisée par évaporation à film essuyé.

3. Procédé selon la revendication 1 ou 2, dans lequel l'évaporateur à film essuyé utilisé dans l'étape c) est un évaporateur à film essuyé à court trajet.

4. Procédé selon l'une quelconque des revendications précédentes, dans lequel l'extrait brut obtenu à l'étape c) est soumis à un fractionnement chromatographique pour produire une fraction de CBD de haute pureté.

5. Procédé selon la revendication 4, dans lequel la fraction de CBD de haute pureté est soumise à une évaporation avant d'être soumise au procédé de cristallisation comprenant les étapes d) et e).

6. Procédé selon la revendication 4 ou 5, dans lequel le fractionnement chromatographique comprend une chromatographie flash, qui est réalisée avec un mélange hexane/acétate d'éthyle comme éluant.

7. Procédé selon l'une quelconque des revendications précédentes, dans lequel l'extrait brut obtenu à l'étape c) est soumis à une hivérisation, pour produire une solution contenant du CBD.

8. Procédé selon la revendication 7, dans lequel la solution contenant du CBD est soumise à une évaporation avant d'être soumise au procédé de cristallisation comprenant les étapes d) et e).

9. Procédé selon l'une quelconque des revendications précédentes, dans lequel le chanvre industriel ou les déchets de chanvre industriel fournis à l'étape a) contiennent moins de 5 %, de préférence moins de 2 %, de CBD en poids de matière sèche.
